# EUROPEAN PATENT APPLICATION

(11) **EP 2 899 546 A1**
(43) Date of publication of application: **29.07.2015**
(21) Application number: 13839384.8
(22) Date of filing: 05.04.2013
(51) Int. Cl.: G01N 35/08, G01N 27/447, G01N 37/00

(54) **ANALYSIS CHIP, AND ANALYSIS DEVICE**

(30) Priority: 19.09.2012 JP 2012206024
(71) Applicant: NEC Corporation, Tokyo 108-8001 (JP)
(72) Inventor: ASOGAWA, Minoru, Tokyo 108-8001 (JP); MISHINA, Yoshinori, Tokyo 108-8001 (JP); IIMURA, Yasuo, Tokyo 108-8001 (JP); HAGIWARA, Hisashi, Tokyo 108-8001 (JP)
(74) Representative: Betten & Resch
(86) International application number: PCT/JP2013/002380
(87) International publication number: WO 2014/045482

(57) **Abstract**

An analysis chip capable of introducing a sample into a capillary without failure and an analysis device using the analysis chip are provided.

An analysis chip (10) according to an exemplary embodiment of the present invention includes a first channel (64) through which a liquid sample flows together with gas; a second channel into which the sample from the first channel (64) is introduced; and a phoresis tank (69) that stores the sample between the first channel (64) and the second channel, at least a bottom surface of the phoresis tank (69) on a side of the second channel being subjected to a treatment to increase a wettability with respect to the liquid.

## Description

### Technical Field

The present invention relates to an analysis chip and an analysis device, and more particularly, to an analysis chip and an analysis device that include a channel through which a sample flows.

### Background Art

Patent literature 1 discloses a microchannel structure in which a porous structure is provided in a part of a microflow channel. A multiphase flow of liquid and gas passes through the porous structure. Patent literature 1 further discloses that the porous structure is subjected to hydrophilizing treatments or hydrophobizing treatments.

### Citation List

### Patent Literature

**Patent Literature** 1: Published Japanese Translation of PCT International Publication for Patent Application, No. 2003-508043

### Summary of Invention

### Technical Problem

In Patent literature 1, however, it is possible that a liquid sample cannot be introduced into a fine channel such as a capillary. Further, even if the liquid sample can be introduced into the channel, air bubbles in the liquid may be mixed into the channel.

The present invention aims to provide an analysis chip and an analysis device capable of introducing a liquid sample into a capillary without failure.

### Solution to Problem

An analysis chip according to one exemplary aspect of the present invention includes: a first channel through which a liquid sample flows; a second channel into which the sample from the first channel is introduced; and a sample tank that stores the sample between the first channel and the second channel, at least a bottom surface of the sample tank on a side of the second channel being subjected to a treatment to increase a wettability with respect to the liquid.

### Advantageous Effects of Invention

According to the present invention, it is possible to provide an analysis chip and an analysis device capable of introducing a liquid sample into a capillary without failure.

### Brief Description of Drawings

Fig. 1 is a view schematically showing each process of a DNA analysis according to an exemplary embodiment of the present invention;
Fig. 2 is a plane view schematically showing a configuration of an analysis chip used for the DNA analysis;
Fig. 3 is a plane view showing a problem when a liquid sample is transferred to a phoresis tank;
Fig. 4 is a plane view showing a part around the phoresis tank provided in the analysis chip according to a first exemplary embodiment;
Fig. 5 is a plane view showing a behavior of air bubbles which have flowed into the phoresis tank;
Fig. 6 is a plane view showing a part around a phoresis tank provided in an analysis chip according to a second exemplary embodiment; and
Fig. 7 is a side view showing a part around a phoresis tank provided in an analysis chip according to other exemplary embodiments.

### Description of Embodiments

With reference to the accompanying drawings, exemplary embodiments of the present invention will be described. The exemplary embodiments described below are examples of the present invention, and the present invention is not limited to the following exemplary embodiments. In this specification and the drawings, the same elements are denoted by the same reference symbols.

### First exemplary embodiment

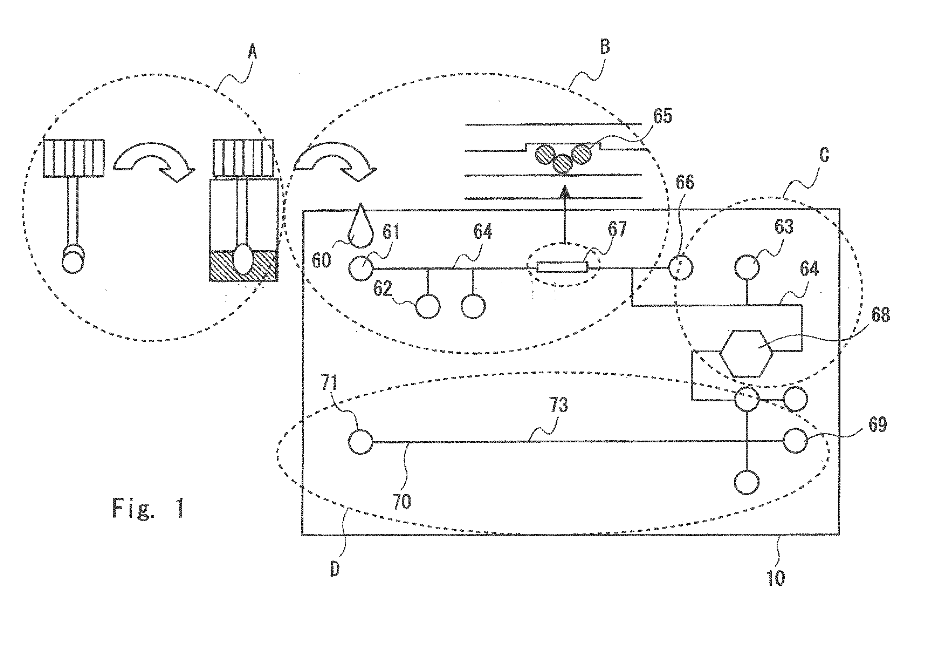

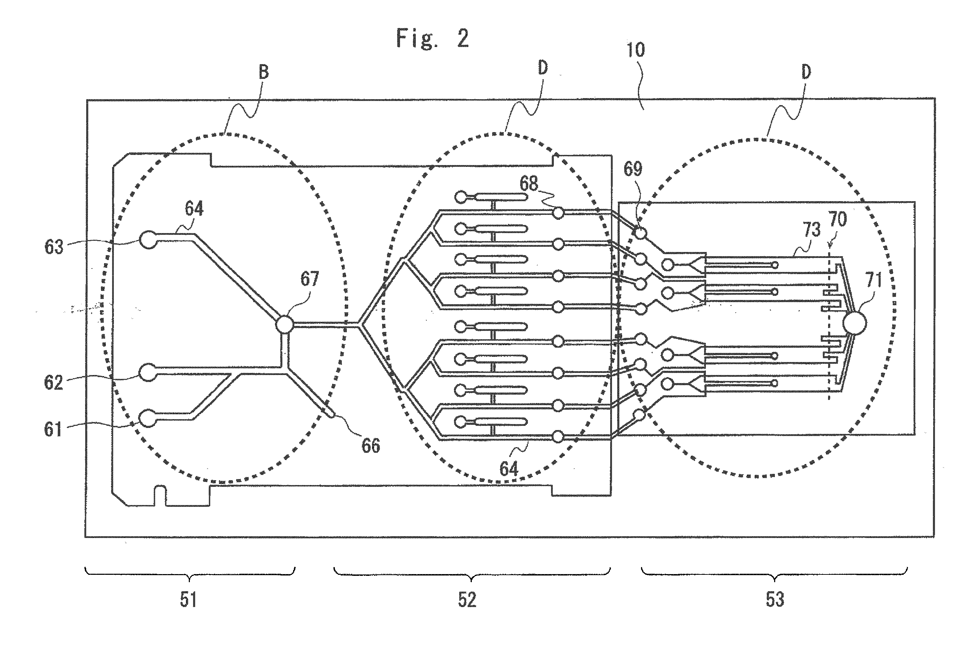

An analysis chip according to an exemplary embodiment is a chip for carrying out a DNA analysis by electrophoresis. With reference to Figs. 1 and 2, a method for carrying out the DNA analysis using the analysis chip will be described below. Fig. 1 is a view schematically showing processes of the DNA analysis using an analysis device according to the exemplary embodiment. Fig. 2 is a plane view schematically showing a configuration of the analysis chip.

As shown in Fig. 1, the DNA analysis method includes a specimen collection process A, a DNA extraction process B, a PCR amplification process C, and an electrophoretic process D. The specimen collection process A is executed in the outside of an analysis chip 10, and the DNA extraction process B, the PCR amplification process C, and the electrophoretic process D are executed in the analysis chip 10.

As shown in Fig. 2, a DNA extraction region 51 in which the DNA extraction process B is executed is provided on an upstream side (left side in Fig. 2) of the analysis chip 10. A PCR amplification region 52 in which the PCR amplification process C is executed is provided on a downstream side (right side in Fig. 2) of the DNA extraction region 51. An electrophoresis region 53 in which the electrophoretic process D is executed is provided on a downstream side of the PCR amplification region 52. DNA, which is a sample, is transferred through the DNA extraction region 51, the PCR amplification region 52, and the electrophoresis region 53 in this order.

In the specimen collection process A, first, a user collects a cell including DNA, which is a sample. The user collects, for example, oral mucosa, blood, or body fluid. Then the cell is disrupted using a reagent to prepare a solution in which DNA is eluted.

In the DNA extraction process B, a solution 60 in which DNA is eluted is injected into the analysis chip 10. The analysis chip 10 includes a sample injection tank 61, a wash solution injection tank 62, a PCR reagent injection tank 63, a channel 64, magnetic beads 65, an outlet 66, a reaction tank 68, a phoresis tank 69, and a phoresis tank 71. The sample injection tank 61, the wash solution injection tank 62, the PCR reagent injection tank 63, the magnetic beads 65, the outlet 66, the reaction tank 68, the phoresis tank 69, and the phoresis tank 71 communicate with one another via the channel 64. That is, the solution 60 is sequentially transferred through the tanks via the channel 64. The channel 64 is narrower than the tanks.

The solution 60 including DNA, which is the sample, is injected into the sample injection tank 61. A wash solution to clean the channel 64 and the like is injected into the wash solution injection tank 62. The solution 60 injected from the sample injection tank 61 is delivered to an extraction part 67 via the channel 64. The magnetic beads 65 to entangle DNA are provided in the extraction part 67. A surface of the magnetic beads 65 has a property of having good compatibility with DNA. Accordingly, by mixing the magnetic beads 65 with the solution 60 in which DNA is eluted, DNA is entangled in the magnetic beads 65. After that, the magnetic beads 65 are cleaned using the wash solution. By using magnets, the magnetic beads 65 can be easily immobilized. Then only DNA is transferred to the next PCR reaction tank 68. Residual wash solution and the like are discharged from the outlet 66.

A PCR reagent that amplifies a specific gene locus is injected into the PCR reagent injection tank 63. The PCR reagent injected into the PCR reagent injection tank 63 is delivered to the PCR reaction tank 68. In the PCR reaction tank 68, DNA is amplified by a polymerase chain reaction (PCR). In this example, as shown in Fig. 2, eight PCR reaction tanks 68 are provided. DNA is then dispensed into the eight PCR reaction tanks 68 together with the PCR reagent. By using two kinds of PCR reagents, 16 (2×8) gene loci can be analyzed at one time.

For example, a temperature control element (not shown) such as a Peltier element is provided immediately below the PCR reaction tank 68. By repeating a predetermined temperature cycle by the temperature control element, only repeated portions of the gene locus can be amplified. Specifically, DNA is PCR-amplified by the temperature control element repeating heating and cooling. Further, the PCR reagent contains fluorescent substances used for labeling. The fluorescent substance used to label DNA may be, for example, 5-FAM, JOE, NED, and ROX. It is thus possible to label a specific base.

The PCR products amplified in the PCR reaction tank 68 are delivered to the phoresis tank 69. The phoresis tank 69 is connected to the phoresis tank 71 via the channel 64. Specifically, the PCR products transferred to the phoresis tank 69 migrate to the phoresis tank 71 via the channel 64. A voltage is applied between the phoresis tank 69 and the phoresis tank 71. Since DNA is negatively charged, DNA migrates to the phoresis tank 71 on the cathode side. The channel 64 between the phoresis tank 69 and the phoresis tank 71 is an extremely thin capillary 73 having a thickness of about 100 µm.

As described above, a voltage is applied between the phoresis tank 69 and the phoresis tank 71. The PCR products labeled by fluorescence are supplied to the capillary and are electrophoresed in gel. In a state in which a voltage is applied by electrophoresis, the migration velocity varies depending on the size of the DNA fragments. The migration distance increases with a decreasing number of bases. It is therefore possible to separate the DNA fragments by size. When PCR products in the capillary are irradiated with excitation light emitted from a light source at a detection position 70 which is between the phoresis tank 69 and the phoresis tank 71, fluorescence is generated from fluorescent substances. The fluorescence generated from the fluorescent substances is spectroscopically measured to obtain observed spectral data. The observed spectral data is obtained for each size of the DNA fragments, or each migration velocity. By analyzing these observed spectral data, it is possible to quantify DNA of a particular sequence and to execute DNA testing.

Further, while the analysis chip is used for DNA testing according to this exemplary embodiment, the analysis chip according to this exemplary embodiment is not limited to being applied to the DNA testing. The analysis chip according to this exemplary embodiment can be applied to various analysis devices. It is possible, for example, to apply the analysis chip to analysis devices which analyze nucleic acid, proteins, compounds and the like. Further, it is possible to label the substances included in the sample by labeled substances other than the fluorescent substances. Further, the analysis may be performed using other methods than spectroscopy.

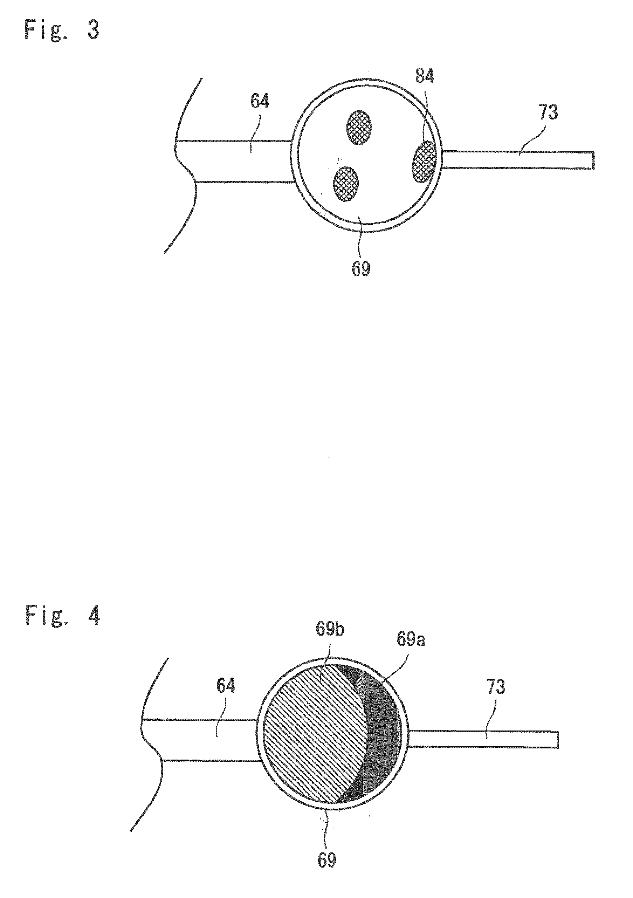

Now, with reference to Fig. 3, a problem that occurs at a connection part between the phoresis tank 69 and the capillary 73 will be described. Fig. 3 is a plane view showing a configuration of a part around the phoresis tank 69. The capillary 73 and the channel 64 having different widths from each other are connected to the phoresis tank 69. A problem in a case in which the sample in the channel 64 is transferred with gas will be described. In the following description, hydrophilic liquid containing the PCR products is used as a sample. The liquid sample is transferred to the phoresis tank 69 with gas.

By supplying gas to the channel 64, liquid which is in the channel 64 is transferred to the phoresis tank 69. At this time, air bubbles 84 flow through the channel 64, which is a first channel, with gas. The air bubbles 84 may stay in the entry side of the capillary 73, which is a second channel. In particular, when the capillary 73 is thinner than the channel 64, the air bubbles 84 block the entrance of the capillary 73. Therefore, even when the voltage is applied, the PCR products, which are the sample, cannot move into the capillary 73, which prevents a correct analysis. Further, the air bubbles 84 may flow into the capillary 73 with the sample. The mixture of the air bubbles 84 with the sample in the minute capillary 73 prevents a correct analysis.

According to this exemplary embodiment, the phoresis tank 69 has the following configuration, which allows the sample to be introduced into the capillary 73 without failure. In the following description, with reference to Fig. 4, the configurations of the phoresis tank 69 and a part around the phoresis tank 69 will be described. Fig. 4 is a plane view showing a configuration of a part around the phoresis tank 69.

The phoresis tank 69 is connected to the channel 64 and the capillary 73. More specifically, the phoresis tank 69 stores the sample between the channel 64 and the capillary 73. The capillary 73 is thinner than the channel 64. The PCR products generated in the PCR reaction tank 68 flow through the channel 64. Specifically, by supplying gas to the channel 64, the sample containing the PCR products flows through the channel 64 with the gas. The sample that flows through the channel 64 reaches the phoresis tank 69. The capillary 73 is not necessarily thinner than the channel 64 as long as the capillary 73 is a channel through which the liquid sample flows.

The phoresis tank 69 is arranged in the upstream side of the electrophoresis device as described above, and a voltage for electrophoresis is applied to the phoresis tank 69. Further, the capillary 73 is provided on the side opposite to the channel 64 with respect to the phoresis tank 69. That is, the channel 64 and the capillary 73 are opposed to each other with respect to the phoresis tank 69. Therefore, the sample that flows through the phoresis tank 69 from the channel 64 flows into the capillary 73.

Further, in this exemplary embodiment, the phoresis tank 69 includes a hydrophilic moiety 69a and a non-hydrophilic moiety 69b. The hydrophilic moiety 69a is formed by performing hydrophilizing treatments on the bottom surface of the phoresis tank 69 on the side of the capillary 73. That is, by making a part of the bottom surface of the phoresis tank 69 hydrophilic, the highly hydrophilic moiety 69a is formed. The hydrophilizing treatments may be performed, for example, by plasma treatments by Reactive Ion Etching (RIE). Alternatively, the hydrophilizing treatments may be performed by applying a hydrophilic organic film or a hydrophilic inorganic film to the bottom surface of the phoresis tank 69. By performing the hydrophilizing treatments, the wettability with respect to the sample becomes high and the contact angle of the sample with respect to the hydrophilic moiety 69a becomes small. In other words, the hydrophilic moiety 69a has a wettability higher than that of the other parts of the analysis chip 10 including the capillary 73 and the channel 64 and the contact angle of the sample becomes small.

Further, in the phoresis tank 69, the bottom surface thereof other than the hydrophilic moiety 69a which was subjected to hydrophilizing treatments is the non-hydrophilic moiety 69b. The hydrophilic moiety 69a is highly hydrophilic compared to the non-hydrophilic moiety 69b. That is, hydrophilicity is distributed on the bottom surface of the phoresis tank 69. In other words, the hydrophilicity of the bottom surface of the phoresis tank 69 is not even. The bottom surface of the phoresis tank 69 may be made uniformly hydrophilic.

The non-hydrophilic moiety 69b has a wettability with respect to the sample lower than that of the hydrophilic moiety 69a and the contact angle of the sample is large. The wettability and the contact angle of the non-hydrophilic moiety 69b are the same as those of the other parts including the capillary 73 and the channel 64. In other words, only the hydrophilic moiety 69a which was subjected to hydrophilizing treatments has a wettability for hydrophilic liquid higher than that of the other parts of the analysis chip 10. When part of the bottom surface of the phoresis tank 69 is made hydrophilic, the hydrophilizing treatments may be performed in a state in which the area of the bottom surface of the phoresis tank 69 which is not subjected to the hydrophilizing treatments is masked.

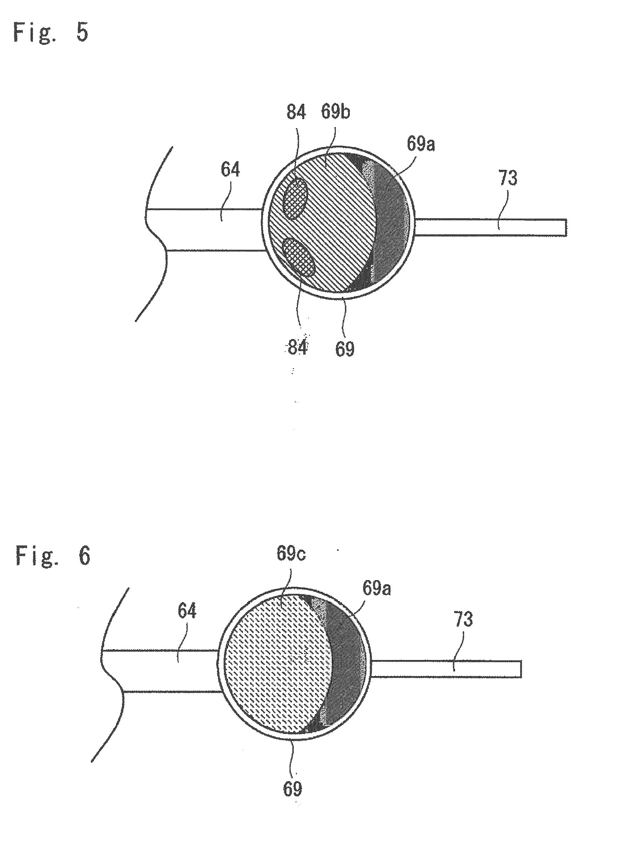

Accordingly, the sample can be introduced to the entry side of the capillary 73 due to the differences in the wettability. Accordingly, as shown in Fig. 5, it is possible to prevent the air bubbles 84 from staying at the entrance of the capillary 73 and to smoothly introduce the sample into the capillary 73. Further, it is possible to prevent the air bubbles from being mixed into the capillary 73. It is therefore possible to introduce the sample into the capillary 73 without failure and to make an accurate analysis.

### Second exemplary embodiment

A configuration of an analysis chip 10 according to this exemplary embodiment will be described with reference to Fig. 6. Fig. 6 is a plane view showing a part around the phoresis tank 69. The second exemplary embodiment is different from the first exemplary embodiment in terms of the configuration of the phoresis tank 69. Since the configurations other than the configuration of the phoresis tank 69 are similar to those of the first exemplary embodiment, the descriptions thereof will be omitted.

In this exemplary embodiment, the phoresis tank 69 includes a hydrophilic moiety 69a and a hydrophobic moiety 69c. That is, the hydrophobic moiety 69c is provided in the phoresis tank 69 in place of the non-hydrophilic moiety 69b. In a bottom surface of the phoresis tank 69, an entry side of the capillary 73 is the hydrophilic moiety 69a and an exit side of the channel 64 is the hydrophobic moiety 69c. The hydrophilic moiety 69a is the same as that in the first exemplary embodiment. In the hydrophobic moiety 69c, hydrophobizing treatments are performed. The hydrophobic moiety 69c may be formed, for example, by applying a hydrophobic organic film or a hydrophobic inorganic film to the bottom surface of the phoresis tank 69. When part of the bottom surface of the phoresis tank 69 is made hydrophobic, the hydrophobizing treatments may be performed using a mask that covers the parts of the bottom surface of the phoresis tank 69 other than the part thereof which is to be the hydrophobic moiety 69c.

The hydrophilic moiety 69a is more hydrophilic than the hydrophobic moiety 69c. That is, hydrophilicity is distributed on the bottom surface of the phoresis tank 69. In other words, the hydrophilicity of the bottom surface of the phoresis tank 69 is not even.

In the hydrophobic moiety 69c that was subjected to the hydrophobizing treatments, a wettability with respect to the sample becomes low and the contact angle of the sample becomes large. The hydrophobic moiety 69c has a wettability lower than that of the other parts of the analysis chip 10 including the capillary 73 and the channel 64. In other words, the contact angle of the sample in the hydrophobic moiety 69c is larger than that of the other parts of the analysis chip 10 including the capillary 73 and the channel 64. Accordingly, the air bubbles 84 are prone to stay in the hydrophobic moiety 69c, whereby the position of the air bubbles 84 can be controlled. It is therefore possible to prevent the air bubbles 84 from staying at the entrance of the capillary 73, whereby the sample can be introduced into the capillary 73 without failure. Further, it is possible to prevent fine air bubbles 84 from being mixed into the capillary 73. It is therefore possible to make a correct analysis.

### Other exemplary embodiments

While the liquid sample is the PCR products in the first and second exemplary embodiments, a liquid sample used in an analysis chip according to this exemplary embodiment is not used for PCR products. While the example in which the bottom surface of the phoresis tank 69 is made hydrophilic has been described above, a side surface of the phoresis tank 69 may be made hydrophilic or hydrophobic. That is, hydrophilicity may be distributed in the phoresis tank 69 in such a way that the air bubbles 84 do not stay in the entry side of the capillary 73.

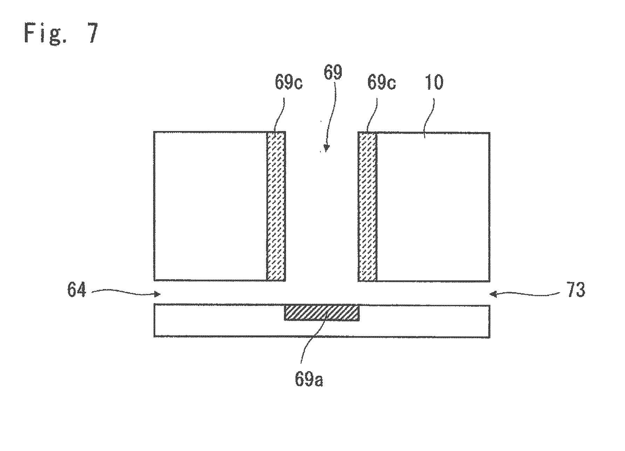

For example, as shown in a side cross-sectional view of Fig. 7, the hydrophilic moiety 69a may be provided by performing hydrophilizing treatments on the whole bottom surface of the phoresis tank 69 and the hydrophobic moieties 69c may be provided by performing hydrophobizing treatments on the side surfaces of the phoresis tank 69. By employing such a configuration, the air bubbles 84 are guided to the upper side of the phoresis tank 69, which prevents the air bubbles 84 from staying at the entrance of the capillary 73. It is therefore possible to introduce the sample into the capillary 73 without failure. Further, also in a case in which the side surface is made hydrophobic, hydrophilicity may be distributed on the bottom surface of the phoresis tank 69. Further, only a part of the side surface of the phoresis tank 69 may be made hydrophobic.

For example, the side surface of the phoresis tank 69 on the side of the channel 64, i.e., the left side surface of Fig. 7, is preferably made hydrophobic. According to such a configuration, the air bubbles 84 can be moved to the phoresis tank 69 on the side of the channel 64, which can prevent the air bubbles 84 from mixed into the capillary 73. Further, the side surface of the phoresis tank 69 on the side of the capillary 73, i.e., the right side surface of Fig. 7, may not be made hydrophobic. Further, the side surface of the phoresis tank 69 on the side of the capillary 73, i.e., the right side surface of Fig. 7 may be made hydrophilic to form the hydrophilic moiety 69a. As described above, the side surface of the phoresis tank 69 on the side of the capillary 73 is made hydrophilic and the side surface of the phoresis tank 69 on the side of the channel 64 is made hydrophobic. It is therefore possible to guide the liquid sample to the capillary 73, whereby the sample can be introduced into the capillary 73 without failure.

While hydrophilicity is distributed in the phoresis tank 69 of the electrophoresis region 53 in the above description, hydrophilicity is distributed in a tank other than the phoresis tank 69. For example, the above configuration may be employed in the sample tank that stores the sample between the channel 64 and the capillary 73. That is, the effects same as those when hydrophilicity is distributed in the phoresis tank 69 of the electrophoresis region 53 are obtained in the sample tank that is connected to the channel through which the liquid sample flows together with gas and is connected to the capillary 73. The analysis device includes the above analysis chip. The analysis device then makes an analysis by migrating the sample in the capillary of the above analysis chip. Therefore, the sample can be accurately analyzed. It is therefore possible to improve the accuracy of the DNA testing.

While gas is supplied to transfer the liquid sample in the above description, hydrophobic liquid may be supplied to transfer the liquid sample. For example, fluid such as oil, which has a wettability different from that of liquid containing the sample, can be supplied to transfer the liquid sample. Oil is hydrophobic and has properties different from those of the hydrophilic liquid. As described above, by supplying oil to the channel 64 together with the liquid sample, the liquid sample is transferred to the phoresis tank 69. At this time, oil, which is more hydrophobic than the liquid sample, does not stay in the hydrophilic moiety 69a. That is, oil is introduced into the non-hydrophilic moiety 69b or the hydrophobic moiety 69c, not into the entry side of the capillary 73, which can prevent oil from flowing into the capillary 73. It is therefore possible to introduce only the liquid sample into the capillary 73 without failure. By supplying fluid such as gas or oil together with the liquid sample, the above described effects can be obtained.

Further, while the hydrophilic liquid has been used as the liquid containing the sample, the liquid containing the sample may instead be hydrophobic liquid. In such a case, if the bottom surface of the phoresis tank 69 in the capillary side is made hydrophobic, the wettability with respect to the sample becomes high in at least the hydrophilic moiety 69a, which causes effects the same as those when the hydrophilic liquid is used. More specifically, the bottom surface of the phoresis tank 69 on the side of at least the capillary 73 is subjected to treatments to increase the wettability with respect to the liquid sample. In the part of the analysis chip 10 corresponding to the hydrophilic moiety 69a, the wettability with respect to the sample liquid becomes higher than that in the other parts of the analysis chip 10. Accordingly, the liquid sample can be introduced into the part of the analysis chip 10 corresponding to the hydrophilic moiety 69a, and the fluid having properties different from those of the sample does not stay in the hydrophilic moiety 69a. Further, the part of the analysis chip 10 corresponding to the hydrophobic moiety 69c may be subjected to treatments to decrease the wettability thereof. Further, the side surface of the phoresis tank 69 may be subjected to treatments to decrease the wettability thereof.

Further, the transfer fluid that flows together with the liquid may either be liquid or gas, and the transfer fluid may either be hydrophilic or non-hydrophilic. Further, only the liquid sample may be transferred without using the transfer fluid. In this case, it is possible to prevent the gas in the phoresis tank 69 from remaining on the side of the capillary 73 as the air bubbles 84 or from being mixed in the capillary 73.

As described above, the wettability of the surface of the phoresis tank 69 can be varied by, for example, plasma treatments or application treatments. Alternatively, the wettability of the phoresis tank 69 may be varied by finely processing the surface of the phoresis tank 69. In one more alternative aspect, the wettability may be varied by using electrowetting that applies an electric field.

While the present invention has been described above with reference to the exemplary embodiments, the present invention is not limited to the above exemplary embodiments. Various changes that can be understood by those skilled in the art may be made on the configurations or the details of the present invention within the scope of the present invention.

This application is based upon and claims the benefit of priority from Japanese Patent Application No. 2012-206024, filed on September 19, 2012, the disclosure of which is incorporated herein in its entirety by reference.

### Industrial Applicability

The analysis chip according to the present invention can be applied to analyze DNA, nucleic acid, proteins, compounds and the like.

### Reference Signs List

- 10: ANALYSIS CHIP
- 51: DNA EXTRACTION REGION
- 52: PCR AMPLIFICATION REGION
- 53: ELECTROPHORESIS REGION
- 60: SOLUTION
- 61: SAMPLE INJECTION TANK
- 62: WASH SOLUTION INJECTION TANK
- 63: PCR REAGENT INJECTION TANK
- 64: CHANNEL
- 65: MAGNETIC BEADS
- 66: OUTLET
- 67: EXTRACTION PART
- 68: PCR REACTION TANK
- 69: PHORESIS TANK
- 69a: HYDROPHILIC MOIETY
- 69b: NON-HYDROPHILIC MOIETY
- 69c: HYDROPHOBIC MOIETY
- 70: DETECTION POSITION

## Claims

1. An analysis chip comprising:
a first channel through which a liquid sample flows;
a second channel into which the sample from the first channel is introduced; and
a sample tank that stores the sample between the first channel and the second channel, at least a bottom surface of the sample tank on a side of the second channel being subjected to a treatment to increase a wettability with respect to the liquid.

2. The analysis chip according to Claim 1, wherein wettability is distributed on the bottom surface of the sample tank.

3. The analysis chip according to Claim 1 or 2, wherein the bottom side of the sample tank on a side of the first channel is subjected to a treatment to decrease the wettability with respect to the liquid.

4. The analysis chip according to any one of Claims 1 to 3, wherein a side surface of the sample tank on a side of the second channel is subjected to a treatment to increase the wettability.

5. An analysis device comprising the analysis chip according to any one of Claims 1 to 4, wherein the analysis device carries out an analysis by migrating the sample in the second channel.
